# EUROPEAN PATENT APPLICATION

(11) **EP 2 871 213 A1**
(43) Date of publication of application: **13.05.2015**
(21) Application number: 13812791.5
(22) Date of filing: 05.07.2013
(51) Int. Cl.: C08L 33/02, A61K 8/19, A61K 8/34, A61K 8/81, A61Q 19/00, C08K 3/16, C08K 5/053

(54) **GEL-LIKE COMPOSITION**

(30) Priority: 05.07.2012 JP 2012151748
(71) Applicant: Sumitomo Seika Chemicals Co., Ltd., Kako-gun, Hyogo 675-0145 (JP)
(72) Inventor: NAKATSUKA, Akio, Himeji-shi Hyogo 672-8076 (JP); MORIMITSU, Yuichiro, Himeji-shi Hyogo 672-8076 (JP); MURAKAMI, Ryosuke, Himeji-shi Hyogo 672-8076 (JP); NISHIKAWA, Yusuke, Himeji-shi Hyogo 672-8076 (JP)
(74) Representative: Vossius & Partner Patentanwälte Rechtsanwälte mbB
(86) International application number: PCT/JP2013/068506
(87) International publication number: WO 2014/007370

(57) **Abstract**

An object of the present invention is to provide a gel-like cosmetic material the viscosity of which is not easily reduced even in the presence of an electrolyte, the cosmetic material ensuring an unconventional jelly-like feel when being applied to the skin, and a smooth sensation after being applied to the skin.

The present invention provides a gel-like composition comprising 0.3 to 3 mass% of an alkyl-modified carboxyl-group-containing water-soluble polymer, 3 to 20 mass% of a polyhydric alcohol, and 0.25 to 5 mass% of an inorganic salt,
the alkyl-modified carboxyl-group-containing water-soluble polymer being
(i) a polymer obtained by polymerizing a (meth)acrylic acid and a C₁₈₋₂₄ alkyl-containing (meth) acrylic acid alkyl ester, or
(ii) a polymer obtained by polymerizing a (meta)acrylic acid, a C₁₈₋₂₄ alkyl-containing (meth)acrylic acid alkyl ester, and a compound having two or more ethylenically unsaturated groups.

## Description

### Technical Field

The present invention relates to a gel-like composition suitable for cosmetics such as skin-care products and hair-care products, the composition comprising an alkyl-modified carboxyl-group-containing water-soluble polymer as a thickener.

### Background Art

As thickeners for cosmetics, natural thickeners such as xanthan gum, semisynthetic thickeners such as hydroxyethyl cellulose, and synthetic thickeners such as carboxyvinyl polymers and alkyl-modified carboxyvinyl polymers have been widely used. In particular, carboxyl-group-containing water-soluble polymers such as carboxyvinyl polymers and alkyl-modified carboxyvinyl polymers are used in various cosmetics because they exhibit excellent thickening properties even when used in small amounts, and can control the feel of cosmetics.

Patent Literature 1, for example, discloses a gel-like cosmetic material comprising 40 to 75 wt% of polyethylene glycol, 20 to 55 wt% of glycerin, and a carboxyvinyl polymer. Patent Literature 2 discloses a cosmetic material for moisturizing, comprising a carboxyvinyl polymer and/or a salt thereof, which may be alkyl-modified, and 15 to 30 mass% of polyhydric alcohol.

Cosmetic materials using such carboxyl-group-containing water-soluble polymers, however, have a problem in that the viscosity sharply decreases in the presence of an electrolyte such as an inorganic salt.

### Citation List

### Patent Literature

PTL 1: JP2001-335429A
PTL 2: JP2010-64986A

### Summary of Invention

### Technical Problem

An object of the present invention is to provide a gel-like composition, the viscosity of which is not reduced even in the presence of an electrolyte, the composition ensuring an unconventional jelly-like feel when being applied to the skin, and a smooth sensation after being applied to the skin.

### Solution to Problem

The present inventors conducted extensive research to attain the above object. As a result, they found that a gel-like composition that maintains its viscosity even in the presence of an electrolyte, has appropriate elasticity when being applied to the skin, and provides a smooth sensation after being applied to the skin can be obtained by using a specific alkyl-modified carboxyl-group-containing water-soluble polymer and a polyhydric alcohol. The inventors conducted further research and have accomplished the present invention.

More specifically, the present invention encompasses the following subject matter:
Item 1.
   A gel-like composition comprising 0.3 to 3 mass% of an alkyl-modified carboxyl-group-containing water-soluble polymer, 3 to 20 mass% of a polyhydric alcohol, and 0.25 to 5 mass% of an inorganic salt,
   the alkyl-modified carboxyl-group-containing water-soluble polymer being
   (i) a polymer obtained by polymerizing a (meth)acrylic acid and a C₁₈₋₂₄ alkyl-containing (meth) acrylic acid alkyl ester, or
   (ii) a polymer obtained by polymerizing a (meta)acrylic acid, a C₁₈₋₂₄ alkyl-containing (meth) acrylic acid alkyl ester, and a compound having two or more ethylenically unsaturated groups.
Item 2.
   The gel-like composition according to Item 1, wherein the alkyl-modified carboxyl-group-containing water-soluble polymer is
   (ii) a polymer obtained by polymerizing a (meth)acrylic acid, a C₁₈₋₂₄ alkyl-containing (meth)acrylic acid alkyl ester, and a compound having two or more ethylenically unsaturated groups.
Item 3.
   The gel-like composition according to Item 2, wherein the alkyl-modified carboxyl-group-containing water-soluble polymer is obtained by polymerizing 100 parts by mass of a (meth) acrylic acid, 0.5 to 5 parts by mass of a C₁₈₋₂₄ alkyl-containing (meth)acrylic acid alkyl ester, and 0.001 to 0.1 parts by weight of a compound having two or more ethylenically unsaturated groups.
Item 4.
   The gel-like composition according to Item 2 or 3, wherein the compound having two or more ethylenically unsaturated groups is at least one member selected from the group consisting of pentaerythritol allyl ethers, diethylene glycol allyl ether, polyethylene glycol diallyl ether, and polyallyl saccharoses.
Item 5.
   The gel-like composition according to any one of Items 1 to 4, wherein the polyhydric alcohol is at least one member selected from the group consisting of 1,3-butylene glycol, glycerin, diglycerine, propylene glycol, sorbitan, and polyethylene glycols.
Item 6.
   The gel-like composition according to any one of Items 1 to 5, wherein 0>b and 0.5>a>0.05 in the formula: tan δ = a x frequency (Hz)^{b}, which indicates a relation of loss tangent (tan δ) and frequency (Hz) in a frequency sweep measurement at a temperature of 25°C and a frequency of 1 to 10 Hz.
Item 7.
   The gel-like composition according to any one of Items 1 to 6, wherein the inorganic salt is an inorganic chloride (preferably at least one member selected from the group consisting of sodium chloride, potassium chloride, and ammonium chloride.)
Item 8.
   The gel-like composition according to any one of Items 1 to 7, wherein the alkyl-modified carboxyl-group-containing water-soluble polymer is such that a neutralized viscous liquid having 1 mass% of the alkyl-modified carboxyl-group-containing water-soluble polymer and a pH of 6.5 to 7.5 has a viscosity of 1500 mPa·s or less, and a transmittance of 90% or more.
Item 9.
   The gel-like composition according to any one of Items 1 to 8, wherein the alkyl-modified carboxyl-group-containing water-soluble polymer is such that the highest viscosity and the transmittance obtained when sodium chloride is added in an amount of 0.25 to 5 parts by mass to 100 parts by mass of the neutralized viscous liquid having 1 mass% of the alkyl-modified carboxyl-group-containing water-soluble polymer and a pH of 6.5 to 7.5, are respectively 15,000 to 40,000 mPa·s and 90% or more.
Item 10.
   The gel-like composition according to any one of Items 1 to 9, wherein the gel-like composition is a gel-like cosmetic material.

### Advantageous Effects of Invention

The gel-like composition of the present invention can be suitably used for cosmetics such as skin-care products and hair-care products because the composition has high viscosity even in the presence of an electrolyte, and has excellent transparency, appropriate elasticity, and jelly-like feel when being applied to the skin, and a smooth sensation after being applied to the skin.

### Brief Description of Drawings

Fig. 1 shows a graph of G''/G'= tan δ (loss tangent) obtained from G'(storage modulus) and G'' (loss modulus) at each frequency, which were obtained by measuring frequency sweep at a frequency of 1 to 10 Hz in Example 5.
Fig. 2 shows a graph indicating the measurement results of frequency dispersion in Example 5 and Comparative Example 6.
Fig. 3 shows a graph indicating the measurement results of strain sweep in Example 5 and Comparative Example 6.

### Description of Embodiments

The gel-like composition of the present invention comprises 0.3 to 3 mass% of a specific alkyl-modified carboxyl-group-containing water-soluble polymer, 3 to 20 mass% of a polyhydric alcohol, and 0.25 to 5 mass% of an inorganic salt. In the present specification, "mass%" indicates "mass/mass%".

The alkyl-modified carboxyl-group-containing water-soluble polymer used in the present invention is preferably (i) a polymer obtained by polymerizing a (meth) acrylic acid and a C₁₈₋₂₄ alkyl-containing (meth)acrylic acid alkyl ester, or (ii) a polymer obtained by polymerizing a (meta) acrylic acid, a C₁₈₋₂₄ alkyl-containing (meth)acrylic acid alkyl ester, and a compound having two or more ethylenically unsaturated groups. In particular, the polymer is preferably obtained by polymerizing 100 parts by mass of a (meth)acrylic acid, 0.5 to 5 parts by mass of a C₁₈₋₂₄ alkyl-containing (meth) acrylic acid alkyl ester, and 0.001 to 0.1 parts by mass of a compound having two or more ethylenically unsaturated groups.

In the present invention, acrylic acids and methacrylic acids are collectively referred to as "(meth)acrylic acids." That is, the expression "(meth)acrylic acid" in the present invention indicates an acrylic acid and/or a methacrylic acid.

The C₁₈₋₂₄ alkyl-containing (meth)acrylic acid alkyl ester indicates an ester of (meth) acrylic acid and C₁₈₋₂₄ alkyl alcohol. Examples include an ester of (meth)acrylic acid and stearyl alcohol, an ester of (meth)acrylic acid and eicosanol, an ester of (meta) acrylic acid and behenyl alcohol, an ester of (meta)acrylic acid and tetracosanol, etc. Of these, stearyl methacrylate, eicosanyl methacrylate, behenyl methacrylate, and tetracosanyl methacrylate are preferably used because the neutralized viscous liquid containing the resulting carboxyl-containing water-soluble polymer, and the neutralized viscous liquid in the presence of an electrolyte have excellent viscometric properties and texture. A commercially available C₁₈₋₂₄ alkyl-containing (meth)acrylic acid alkyl ester can be used, and usable examples include Blemmer VMA70 (trade name, C₁₈₋₂₄ alkylmethacrylate produced by NOF Corporation). The C₁₈₋₂₄ alkyl-containing (meth)acrylic acid alkyl esters can be used singly or in a combination of two or more.

The amount of the C₁₈₋₂₄ alkyl-containing (meth) acrylic acid alkyl ester in the alkyl-modified carboxyl-group-containing water-soluble polymer is preferably 0.5 to 5 parts by mass, and more preferably 1 to 3 parts by mass per 100 parts by mass of the (meth) acrylic acid. When the amount of the C₁₈₋₂₄ alkyl-containing (meth) acrylic acid alkyl ester is 0.5 parts by mass or more per 100 parts by mass of the (meth) acrylic acid, the thickening properties of a composition using the polymer are improved. In contrast, when the amount of the C₁₈₋₂₄ alkyl-containing (meth)acrylic acid alkyl ester is 5 parts by mass or less, a reduction in the transmittance of the neutralized viscous liquid containing the resulting alkyl-modified carboxyl-group-containing water-soluble polymer can be further decreased. In particular, when the alkyl-modified carboxyl-group-containing water-soluble polymer is used as a cosmetic component, the transmittance of the neutralized viscous liquid is also important in order to use the polymer in various applications.

The neutralized viscous liquid used herein is a liquid obtained by dissolving the alkyl-modified carboxyl-group-containing water-soluble polymer in water, and then neutralizing the mixture with a pH adjuster. Examples of the pH adjuster used herein include alkalis including alkali metal hydroxides such as sodium hydroxide, amines such as triethanolamine and diisopropanolamine, etc. In the present specification, "neutralize" means adjusting the pH to 6.5 to 7.5.

The compound having two or more ethylenically unsaturated groups is optionally used to obtain the alkyl-modified carboxyl-group-containing water-soluble polymer. The compound having two or more ethylenically unsaturated groups is not limited, and preferable examples include pentaerythritol allyl ethers, diethylene glycol diallyl ether, polyethylene glycol diallyl ether, polyallyl saccharoses, etc. Examples of the pentaerythritol allyl ethers include pentaerythritol diallyl ether, pentaerythritol triallyl ether, and pentaerythritol tetraallyl ether. Of these, pentaerythritol tetraallyl ether is preferable. The compounds having two or more ethylenically unsaturated groups can be used singly or in a combination of two or more.

In the present invention, the amount of the compound having two or more ethylenically unsaturated groups is preferably 0.001 to 0.1 parts by mass, and more preferably 0.001 to 0.022 parts by mass per 100 parts by mass of the (meth)acrylic acid. When the amount of the compound having two or more ethylenically unsaturated groups is 0.1 parts by mass or less, the gel-like composition containing the resulting alkyl-modified carboxyl-group-containing water-soluble polymer, which is used as a cosmetic material, can attain more preferable jelly-like feel specific to the present invention.

In the present invention, the method for obtaining an alkyl-modified carboxyl-group-containing water-soluble polymer by polymerizing a (meth) acrylic acid and a C₁₈₋₂₄ alkyl-containing (meth)acrylic acid alkyl ester, and optionally, a compound having two or more ethylenically unsaturated groups (i.e., method for obtaining the polymer (i) or (ii) above) is not particularly limited. Conventional methods can be used in which these starting materials are stirred in a solvent under an inactive gas atmosphere and polymerized using a polymerization initiator.

Examples of the inactive gas to obtain an inactive gas atmosphere include nitrogen gas, argon gas, etc.

The solvent dissolves a (meth)acrylic acid, a C₁₈₋₂₄ alkyl-containing (meth)acrylic acid alkyl ester, and a compound having two or more ethylenically unsaturated groups. However, the solvent preferably does not dissolve the resulting alkyl-modified carboxyl-group-containing water-soluble polymer and does not inhibit the reaction. Examples of the solvent include hydrocarbon solvents, ester-based solvents, etc. Examples of the hydrocarbon solvents include normal pentane, normal hexane, normal heptane, cyclopentane, cyclohexane, etc. Examples of the ester-based solvents include methyl acetate, ethyl acetate, propyl acetate, butyl acetate, etc. Of these, normal hexane, normal heptane, and ethyl acetate are preferable. The solvents can be used singly or in a combination of two or more (i.e., as a mixture solvent). Although the amount of the solvent is not particularly limited, it is preferably 300 to 5,000 parts by mass per 100 parts by mass of the (meth) acrylic acid from the viewpoint of improvement in stirring operation and economical efficiency.

The polymerization initiator is preferably a radical polymerization initiator. Examples include α-α'-azoisobutyronitrile, 2,2'-azobis-2,4-dimethylvaleronitrile, 2,2'-azobis methyl isobutyrate, etc. Of these, 2,2'-azobis methyl isobutyrate is preferably used because an alkyl-modified carboxyl-group-containing water-soluble polymer having a high molecular weight can be obtained.

The amount of the polymerization initiator can be suitably determined so that the polymerization reaction can proceed. The amount is not particularly limited, but it is desirably 0.00003 to 0.002 mol per mol of the (meth)acrylic acid.

The reaction temperature can be suitably determined. For example, it is preferably 50 to 90°C, and more preferably 55 to 75°C. The reaction time depends on the reaction temperature, and thus is not limited; however, it is typically about 0.5 to 5 hours.

After the completion of the reaction, the reaction solution may be, for example, heated to 80 to 130°C to volatize the solvent for removal, thereby obtaining an alkyl-modified carboxyl-group-containing water-soluble polymer.

The alkyl-modified carboxyl-group-containing water-soluble polymer is preferably such that the neutralized viscous liquid (pH of 6.5 to 7.5) having 1 mass% of the polymer has a viscosity of 1,500 mPA·s or less, and a transmittance of 90% or more. Also, the alkyl-modified carboxyl-group-containing water-soluble polymer is preferably such that the highest viscosity obtained when 0.25 to 5 parts by mass of sodium chloride is added to 100 parts by mass of the neutralized viscous liquid is 15,000 to 40,000 mPa·s, and the transmittance is 90% or more.

"Highest viscosity obtained when 0.25 to 5 parts by mass of sodium chloride is added" indicates the viscosity of a solution that has the highest viscosity of eight solutions obtained by adding 0.25, 0.5, 0.75, 1, 2, 3, 4, or 5 parts by mass of sodium chloride to 100 parts by mass of the neutralized viscous liquid.

The transmittance herein is the value obtained as follows. After performing deaeration in a centrifuge at 2,000 rpm for five minutes, the transmittance is measured at a measurement wavelength of 425 nm using a 1-cm path length cell (i.e., the rate of transmittance at 425 nm when the transmittance of pure water is defined as 100%.)

The viscosity herein is measured by using a BH-type rotational viscometer under the conditions of 25°C and a spindle rotor No. 6 rotational speed of 20 rpm for one minute.

Examples of the polyhydric alcohol used in the present invention include 1,3-butylene glycol, glycerin, diglycerin, propylene glycol, sorbitan, polyethylene glycol, maltitol, etc. In particular, the use of glycerin, 1,3-butylene glycol, and propylene glycol are preferable. The polyhydric alcohols can be used singly or in a combination of two or more.

Examples of the inorganic salt used in the present invention include inorganic chlorides. Examples of the inorganic chlorides include sodium chloride, potassium chloride, ammonium chloride, etc. Of these, sodium chloride can be preferably used. The inorganic salts can be used singly or in a combination of two or more.

As described above, the gel-like composition of the present invention includes an inorganic salt; however, since the inorganic salt is an electrolyte, it may be ionized and present as ion in the gel-like composition. The gel-like composition of the present invention includes a gel-like composition that contains an inorganic salt in such a state.

The gel-like composition of the present invention can be obtained, for example, by dissolving a specific alkyl-modified carboxyl-group-containing water-soluble polymer, a polyhydric alcohol, and an inorganic salt in water so that the alkyl-modified carboxyl-group-containing water-soluble polymer, polyhydric alcohol, and inorganic salt are respectively contained in an amount of 0.3 to 3 mass%, 3 to 20 mass%, and 0.25 to 5 mass%. The manner and order of the dissolution of these components are not particularly limited. For example, the alkyl-modified carboxyl-group-containing water-soluble polymer is first dissolved in water, then the polyhydric alcohol, and optionally an alkali or alkali metal hydroxide for neutralization are added, and then the inorganic salt is added. Examples of the alkali or alkali metal hydroxide optionally added for neutralization include sodium hydroxide.

The amount of the alkyl-modified carboxyl-group-containing water-soluble polymer in the gel-like composition of the present invention is, as described above, 0.3 to 3 mass%, preferably 0.5 to 2 mass%, and more preferably 0.5 to 1.5 mass% based on the entire composition.

The amount of the polyhydric alcohol is, as described above, 3 to 20 mass%, and preferably 5 to 15 mass% based on the entire composition.

The amount of the inorganic salt is, as described above, 0.25 to 5 mass%, and preferably 0.3 to 4 mass% based on the entire composition.

As described above, the gel-like composition of the present invention has excellent viscosity even in the presence of an electrolyte and excellent transparency, and ensures appropriate elasticity and jelly-like feel when being applied to the skin, and a smooth sensation after being applied to the skin. Such a feel and tactile sensation are due to the specific properties of the gel-like composition of the present invention. In particular, the gel-like composition of the present invention preferably satisfies (1) 0>b and 0.5>a>0.05 in the formula: tan δ = a x frequency (Hz)^{b}, which indicates the relation between the frequency(Hz) and the loss tangent (tan δ) obtained in the frequency sweep measurement at a temperature of 25°C and a frequency of 1 to 10 Hz.

The loss tangent (tan δ) is defined as the rate (G''/G') of the storage modulus (G') to the loss modulus (G''), and is used as one of the indices to evaluate properties such as viscoelasticity. The higher the loss tangent, the lower the rebound resilience. For example, the loss tangent is used as an index of sol or gel; tan δ > 1 typically indicates a sol.

In the present invention, it was found that a particularly preferable feel and tactile sensation can be obtained when 0>b and 0.5>a>0.05 in the formula (tan δ = a x frequency (Hz)^{b}). When the conditions of *a* (0.5>a>0.05) are satisfied, b is preferably -0.2 or less, more preferably -0.25 or less, and even more preferably -0.3 or less. When the conditions of b (0>b) are satisfied, a is preferably 0.1 to 0.4, more preferably 0.15 to 0.45, and even more preferably 0.2 to 0.45. It is particularly preferable that the conditions of *a* and b be satisfied in combination.

In particular, preferable feel and tactile sensation, which are specific to the present invention, are obtained presumably because b is a negative number (preferably -0.2 or less).

The gel-like composition of the present invention preferably exhibits physical properties (2) and/or (3) below.
(2) The viscosity is 10,000 mPa·s or more.
(3) The linear strain is 30% or more.

The viscosity of item (2) above is measured by using a BH-type rotational viscometer under the conditions of 25°C and a spindle rotor No. 6 rotational speed of 20 rpm for one minute. The viscosity is preferably 12,000 to 40,000 mPa·s, and more preferably 14,000 to 30,000 mPa·s.

The linear strain (%) of item (3) above is obtained by using a strain-sweep measurement at a frequency of 1 Hz.

The gel-like composition of the present invention can include other components as long as the effect of the present invention is not impaired. For example, components typically contained in cosmetics can be incorporated. Examples of such components include minerals, other thickeners, alcohols, pH adjusters, moisturizers, oil solutions, salts, anionic surfactants, nonionic surfactants, cationic surfactants, chelating agents, antiseptics, antioxidants, ultraviolet ray absorbents, pigments, flavoring agents, etc. These components can be suitably used for preparing the gel-like composition. The specific form of the gel-like composition used as a cosmetic material is not particularly limited. For example, the gel-like composition of the present invention can be used as cosmetics such as lotion, milky lotion, serum, cream pack, massage cream, gel cream, cleansing cream, cleansing gel, cleansing foam, sunscreen, styling gel, eye liner, mascara, lip, and foundation.

### Examples

The present invention is explained in detail with reference to the Examples and Comparative Examples; however, the present invention is not limited to or by these.

### Synthesis of Alkyl-modified Carboxyl-group-containing Water-soluble Polymer

### Production Example 1

Acrylic acid (45 g, 0.625 mol), Blemmar VMA70 (0.45 g), which is a C₁₈₋₂₄ alkyl-containing (meth) acrylic acid alkyl ester produced by NOF Corporation (a mixture of 10 to 20 parts by mass of stearyl methacrylate, 10 to 20 parts by mass of eicosanyl methacrylate, 59 to 80 parts by mass of behenyl methacrylate, and 1 part by mass or less of tetracosanyl methacrylate), normal hexane (150 g), and 2,2'-azobis methyl isobutyrate (0.081 g, 0.00035 mol) were introduced into a four-necked flask (500 mL) equipped with a stirrer, a thermometer, a nitrogen-blowing tube, and a condenser tube. Subsequently, the mixture was uniformly stirred and mixed, and then nitrogen gas was blown into the solution to remove oxygen present in the upper space of the reaction container, and in the starting materials and solvent. Subsequently, the resultant was maintained at 60 to 65°C under a nitrogen atmosphere, and reacted for four hours. After the completion of the reaction, the resulting slurry was heated to 90°C to distill off the normal hexane, and drying under reduced pressure was performed at 110°C and 10 mmHg for eight hours, thereby obtaining 43 g of a fine white powdery alkyl-modified carboxyl-group-containing water-soluble polymer (Polymer 1).

### Production Example 2

The procedure was performed in a similar manner as in Production Example 1, except that the amount of Blemmar VMA70 (produced by NOF Corporation) was changed from 0.45 g to 1.35 g, thus obtaining 44 g of a fine white powdery alkyl-modified carboxyl-group-containing water-soluble polymer (Polymer 2).

### Production Example 3

The procedure was performed in a similar manner as in Production Example 1, except that the amount of Blemmar VMA70 (produced by NOF Corporation) was changed from 0.45 g to 2.25 g, thus obtaining 45 g of a fine white powdery alkyl-modified carboxyl-group-containing water-soluble polymer (Polymer 3).

### Production Example 4

The procedure was performed in a similar manner as in Production Example 1, except that 0.02 g of pentaerythritol allyl ether was used in addition to acryl acid (45 g, 0.625 mol), Blemmar VMA70 (produced by NOF Corporation), normal hexane (150 g), and 2,2'-azobis methyl isobutyrate (0.081 g, 0.00035 mol), thus obtaining 43 g of a fine white powdery alkyl-modified carboxyl-group-containing water-soluble polymer (Polymer 4).

### Production Example 5

The procedure was performed in a similar manner as in Production Example 4, except that the amount of Blemmar VMA70 (produced by NOF Corporation) was changed from 0.45 g to 1.35 g, thus obtaining 44 g of a fine white powdery alkyl-modified carboxyl-group-containing water-soluble polymer (Polymer 5).

### Production Example 6

The procedure was performed in a similar manner as in Production Example 4, except that the amount of Blemmar VMA70 (produced by NOF Corporation) was changed from 0.45 g to 2.25 g, thus obtaining 45 g of a fine white powdery alkyl-modified carboxyl-group-containing water-soluble polymer (Polymer 6).

### Production Example 7

Acrylic acid (45 g, 0.625 mol), pentaerythritol allyl ether (0.02 g), normal hexane (150 g), and 2,2'-azobis methyl isobutyrate (0.081 g, 0.00035 mol) were introduced into a four-necked flask (500 mL) equipped with a stirrer, a thermometer, a nitrogen-blowing tube, and a condenser tube. Subsequently, the mixture was uniformly stirred and mixed, and then nitrogen gas was blown into the solution to remove oxygen present in the upper space of the reaction container, and in the starting materials and solvent. Subsequently, the resultant was maintained at 60 to 65°C under a nitrogen atmosphere, and reacted for four hours. After the completion of the reaction, the resulting slurry was heated to 90°C to distill off the normal hexane, and drying under reduced pressure was performed at 110°C and 10 mmHg for eight hours, thereby obtaining 42 g of a fine white powdery carboxyl-group-containing water-soluble polymer (Polymer 7).

### Production Example 8

The procedure was performed in a similar manner as in Production Example 7, except that the amount of pentaerythritol allyl ether was changed from 0.02 g to 0.27 g, thus obtaining 42 g of a fine white powdery carboxyl-group-containing water-soluble polymer (Polymer 8).

The pentaerythritol allyl ether used in all of the Production Examples was pentaerythritol tetra allyl ether.

### Evaluation of Alkyl-modified Carboxyl-group-containing Waster-soluble Polymer

To evaluate the properties as thickeners of Polymers 1 to 8 obtained in Production Examples 1 to 8, neutralized viscous liquids having 1 mass% of Polymers 1 to 8, and electrolyte-added solutions prepared by adding sodium chloride to each of the neutralized viscous liquids were evaluated for viscosity and transmittance.

### (1) Preparation of Test Sample

Polymers in the predetermined number used as test samples were weighed in an amount of three grams each, and each of the polymers was gradually added to deinonized water (280 g) while stirring. Subsequently, 6 wt% sodium hydroxide (17 g) was further added to each mixture while stirring to prepare uniform solutions, thus obtaining neutralized viscous liquids each having 1 mass% of a polymer. These neutralized viscous liquids had a pH of 6.5 to 7.5. Separately, sodium chloride (0.75, 1.5, 2.25, 3, 6, 9, 12, or 15 g) was added to each of the neutralized viscous liquids (300 g), followed by stirring and dissolution, thereby preparing electrolyte-added solutions having different sodium chloride concentrations. The 1 mass% neutralized viscous liquids, and the electrolyte-added solutions to which sodium chloride was added, were allowed to stand for one hour, and used as test samples in the evaluation below.

### (2) Viscosity Measurement

The viscosity of each test sample was measured by using a BH-type rotational viscometer under the conditions of 25°C and a spindle rotor No. 6 rotational speed of 20 rpm for one minute. Tables 1 to 4 show the measurement results.

### (3) Transmittance Measurement

After each test sample was deaerated in a centrifuge at 2,000 rpm for five minutes, the transmittance was measured at a measurement wavelength of 425 nm using a 1-cm path length cell. The sample was typically considered transparent in visual observation when the transmittance was 90% or more. Tables 1 to 4 show the measurement results.

**Table 1**

| NaCl amount (g/100 g neutralized viscous liquid) | Polymer 1 | | | Polymer 2 | | |
|---|---|---|---|---|---|---|
| | Viscosity (mPa·s) | Transmittance (%) | Note | Viscosity (mPa·s) | Transmittance (%) | Note |
| 0 | 100 | 99 | | 270 | 98 | |
| 0.25 | 950 | 98 | | 10350 | 98 | |
| 0.5 | 1650 | 98 | | 15900 | 98 | |
| 0.75 | 2100 | 96 | | 15000 | 98 | |
| 1 | 1000 | 95 | | 14500 | 97 | |
| 2 | 700 | 94 | | 13350 | 94 | |
| 3 | 5000 | 92 | | 3950 | 60 | |
| 4 | 15000 | 90 | | 400 | - | Phase separation |
| 5 | 10750 | 88 | | 50 | - | Phase separation |

**Table 2**

| NaCl amount (g/100 g neutralized viscous liquid) | Polymer 3 | | | Polymer 4 | | |
|---|---|---|---|---|---|---|
| | Viscosity (mPa·s) | Transmittance (%) | Note | Viscosity (mPa·s) | Transmittance (%) | Note |
| 0 | 900 | 99 | | 110 | 99 | |
| 0.25 | 25850 | 98 | | 1000 | 98 | |
| 0.5 | 20750 | 96 | | 1800 | 98 | |
| 0.75 | 17800 | 94 | | 2250 | 98 | |
| 1 | 14500 | 92 | | 1500 | 95 | |
| 2 | 3600 | 70 | Phase separation | 900 | 94 | |
| 3 | - | - | Phase separation | 5100 | 92 | |
| 4 | - | - | Phase separation | 15250 | 90 | |
| 5 | - | - | | 10400 | 87 | |

**Table 3**

| NaCl amount (g/100 g neutralized viscous liquid) | Polymer 5 | | | Polymer 6 | | |
|---|---|---|---|---|---|---|
| | Viscosity (mPa·s) | Transmittance (%) | Note | Viscosity (mPa·s) | Transmittance (%) | |
| 0 | 470 | 98 | | 1500 | 99 | |
| 0.25 | 10300 | 98 | | 26000 | 98 | |
| 0.5 | 16000 | 98 | | 20500 | 95 | |
| 0.75 | 15500 | 97 | | 18000 | 94 | |
| 1 | 16000 | 96 | | 13500 | 92 | |
| 2 | 15050 | 94 | | 2800 | 68 | |
| 3 | 7850 | 60 | | - | - | Phase separation |
| 4 | 900 | 37 | | - | - | Phase separation |
| 5 | 50 | - | Phase separation | - | - | Phase separation |

**Table 4**

| NaCl amount (g/100 g neutralized viscous liquid) | Polymer 7 | | | Polymer 8 | | |
|---|---|---|---|---|---|---|
| | Viscosity (mPa·s) | Transmittance (%) | Note | Viscosity (mPa·s) | Transmittance (%) | Note |
| 0 | 5500 | 99 | | 81000 | 96 | |
| 0.25 | 2000 | 99 | | 30000 | 70 | |
| 0.5 | 1000 | 98 | | 14200 | 62 | |
| 0.75 | 300 | 98 | | 8300 | 54 | |
| 1 | 100 | 97 | | 5120 | 31 | |
| 2 | 50 | 96 | | 1500 | 11 | |
| 3 | 50 | 93 | | 660 | 2 | |
| 4 | 50 | 92 | | | | |
| 5 | 50 | 89 | | | | |

### Example 1

The alkyl-modified carboxyl-group-containing water-soluble polymer (2 g) obtained in Production Example 1 was gradually added to pure water (deionized water, 98 g) while stirring to uniformly disperse the polymer, thereby obtaining a 2 mass% aqueous solution. Pure water (50 g); and 1,3-butylene glycol (5 g) and glycerin (5 g) used as polyhydric alcohols were added to the resulting aqueous solution (100 g) to obtain a uniform mixture, and then a 6 mass% sodium hydroxide aqueous solution (9 g) was added thereto. An aqueous solution (31 g) containing sodium chloride (8 g) (NaCl concentration: 4 mass%) was further added, thereby preparing a gel-like composition.

### Example 2

The alkyl-modified carboxyl-group-containing water-soluble polymer (2 g) obtained in Production Example 2 was gradually added to pure water (deionized water, 98 g) while stirring to uniformly disperse the polymer, thereby obtaining a 2 mass% aqueous solution. Pure water (50 g), and 1,3-butylene glycol (5 g) and glycerin (5 g) used as polyhydric alcohols were added to the resulting aqueous solution (100 g) to obtain a uniform mixture, and then a 6 mass% sodium hydroxide aqueous solution (9 g) was added thereto. An aqueous solution (31 g) containing sodium chloride (1 g) (NaCl concentration: 0.5 mass%) was further added, thereby preparing a gel-like composition.

### Example 3

The alkyl-modified carboxyl-group-containing water-soluble polymer (2 g) obtained in Production Example 3 was gradually added to pure water (deionized water, 98 g) while stirring to uniformly disperse the polymer, thereby obtaining a 2 mass% aqueous solution. Pure water (50 g), and 1,3-butylene glycol (5 g) and glycerin (5 g) used as polyhydric alcohols were added to the resulting aqueous solution (100 g) to obtain a uniform mixture, and then a 6 mass% sodium hydroxide aqueous solution (9 g) was added thereto. An aqueous solution (31 g) containing sodium chloride (0.5 g) (NaCl concentration: 0.25 mass%) was further added, thereby preparing a gel-like composition.

### Example 4

The alkyl-modified carboxyl-group-containing water-soluble polymer (2 g) obtained in Production Example 4 was gradually added to pure water (deionized water, 98 g) while stirring to uniformly disperse the polymer, thereby obtaining a 2 mass% aqueous solution. Pure water (50 g), and 1,3-butylene glycol (5 g) and glycerin (5 g) used as polyhydric alcohols were added to the resulting aqueous solution (100 g) to obtain a uniform mixture, and then a 6 mass% sodium hydroxide aqueous solution (9 g) was added thereto. An aqueous solution (31 g) containing sodium chloride (8 g) (NaCl concentration: 4 mass%) was further added, thereby preparing a gel-like composition.

### Example 5

The alkyl-modified carboxyl-group-containing water-soluble polymer (2 g) obtained in Production Example 5 was gradually added to pure water (deionized water, 98 g) while stirring to uniformly disperse the polymer, thereby obtaining a 2 mass% aqueous solution. Pure water (50 g), and 1,3-butylene glycol (5 g) and glycerin (5 g) used as polyhydric alcohols were added to the resulting aqueous solution (100 g) to obtain a uniform mixture, and then a 6 mass% sodium hydroxide aqueous solution (9 g) was added thereto. An aqueous solution (31 g) containing sodium chloride (1 g) (NaCl concentration: 0.5 mass%) was further added, thereby preparing a gel-like composition.

### Example 6

The alkyl-modified carboxyl-group-containing water-soluble polymer (2 g) obtained in Production Example 6 was gradually added to pure water (deionized water, 98 g) while stirring to uniformly disperse the polymer, thereby obtaining a 2 mass% aqueous solution. Pure water (50 g), and 1,3-butylene glycol (5 g) and glycerin (5 g) used as polyhydric alcohols were added to the resulting aqueous solution (100 g) to obtain a uniform mixture, and then a 6 mass% sodium hydroxide aqueous solution (9 g) was added thereto. An aqueous solution (31 g) containing sodium chloride (0.5 g) (NaCl concentration: 0.25 mass%) was further added, thereby preparing a gel-like composition.

### Comparative Example 1

The alkyl-modified carboxyl-group-containing water-soluble polymer (2 g) obtained in Production Example 4 was gradually added to pure water (deionized water, 98 g) while stirring to uniformly disperse the polymer, thereby obtaining a 2 mass% aqueous solution. Pure water (60 g) was added to the resulting aqueous solution (100 g) to obtain a uniform mixture, and then a 6 mass% sodium hydroxide aqueous solution (9 g) was added thereto. An aqueous solution (31 g) containing sodium chloride (8 g) (NaCl concentration: 4 mass%) was further added, thereby preparing a gel-like composition.

### Comparative Example 2

The alkyl-modified carboxyl-group-containing water-soluble polymer (2 g) obtained in Production Example 5 was gradually added to pure water (deionized water, 98 g) while stirring to uniformly disperse the polymer, thereby obtaining a 2 mass% aqueous solution. Pure water (60 g) was added to the resulting aqueous solution (100 g) to obtain a uniform mixture, and then a 6 mass% sodium hydroxide aqueous solution (9 g) was added thereto. An aqueous solution (31 g) containing sodium chloride (1 g) (NaCl concentration: 0.5 mass%) was further added, thereby preparing a gel-like composition.

### Comparative Example 3

The alkyl-modified carboxyl-group-containing water-soluble polymer (2 g) obtained in Production Example 6 was gradually added to pure water (deionized water, 98 g) while stirring to uniformly disperse the polymer, thereby obtaining a 2 mass% aqueous solution. Pure water (60 g) was added to the resulting aqueous solution (100 g) to obtain a uniform mixture, and then a 6 mass% sodium hydroxide aqueous solution (9 g) was added thereto. An aqueous solution (31 g) containing sodium chloride (0.5 g) (NaCl concentration: 0.25 mass%) was further added, thereby preparing a gel-like composition.

### Comparative Example 4

The polymer (2 g) obtained in Production Example 6 as a carboxyl-group-containing water-soluble polymer (2 g) was gradually added to pure water (deionized water, 98 g) while stirring to uniformly disperse the polymer, thereby obtaining a 2 mass% aqueous solution. Pure water (81 g), and 1,3-butylene glycol (5 g) and glycerin (5 g) used as polyhydric alcohols were added to the resulting aqueous solution (100 g) to obtain a uniform mixture, and then a 6 mass% sodium hydroxide aqueous solution (9 g) was added, thereby preparing a gel-like composition.

### Comparative Example 5

The polymer (2 g) obtained in Production Example 7 as a carboxyl-group-containing water-soluble polymer (2 g) was gradually added to pure water (deionized water, 98 g) while stirring to uniformly disperse the polymer, thereby obtaining a 2 mass% aqueous solution. Pure water (50 g), and 1,3-butylene glycol (5 g) and glycerin (5 g) used as polyhydric alcohols were added to the resulting aqueous solution (100 g) to form a uniform mixture, and then a 6 mass% sodium hydroxide aqueous solution (9 g) was added thereto. An aqueous solution (31 g) containing sodium chloride (1 g) (NaCl concentration: 0.5 mass%) was further added, thereby preparing a gel-like composition.

### Comparative Example 6

The polymer (2 g) obtained in Production Example 8 as a carboxyl-group-containing water-soluble polymer was gradually added to pure water (deionized water, 98 g) while stirring to uniformly disperse the polymer, thereby obtaining a 2 mass% aqueous solution. Pure water (85.5 g), and 1,3-butylene glycol (5 g) and glycerin (5 g) used as polyhydric alcohols were added to the resulting aqueous solution (100 g) to form a uniform mixture, and then a 6 mass% sodium hydroxide aqueous solution (4.5 g) was added, thereby preparing a gel-like composition.

The gel-like compositions obtained in Examples 1 to 6 and Comparative Examples 1 to 6 were measured for viscosity, rheology, jelly-like feel, and smoothness. However, the rheology of Comparative Examples 4 and 5 could not be measured because the compositions had low viscosity and did not become gel-like compositions.

### (1) Viscosity Measurement

The viscosity of each test sample was measured using a BH-type rotational viscometer under the conditions of 25°C and a spindle rotor No. 6 rotational speed of 20 rpm for one minute.

### (2) Rheology Measurement

The frequency sweep at a frequency of 1 to 10 Hz and the strain sweep at a frequency of 1 Hz of each test sample were measured using a commercially available viscoelastic measurement device (rheometer).

### Measurement Conditions

Rheometer: AR-2000ex produced by TA instruments
Plate: 60 mm, 4° cone plate
Measurement temperature: 25°C
Strain-sweep measurement: 0.1 to 1,000% (1 Hz)
Frequency-sweep measurement: 10 to 1 Hz (strain: 0.1%)

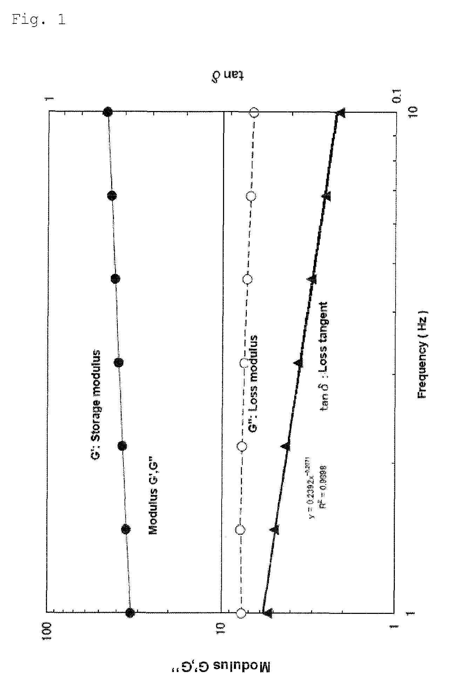

As shown in Fig. 1, G''/G' = tan δ (loss tangent) can be obtained from G' (storage modulus) and G'' (loss modulus) measured at each frequency. Fig. 1 shows the measurement results of Example 5.

Regarding the tan δ and frequency (Hz), *a* and b were calculated from the power approximation formula: tan δ = a x frequency (Hz)^{b}.

Taking the following sensory evaluation results also into consideration, the composition ensuring both preferable jelly-like feel and smoothness was considered to be within the range of 0>b and 0.5>a>0.05.

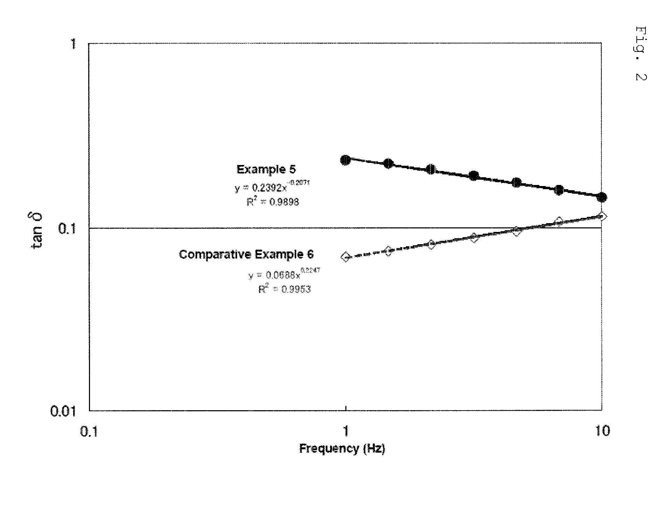

Fig. 2 shows the examples of frequency sweep measured in Example 5 and Comparative Example 6.

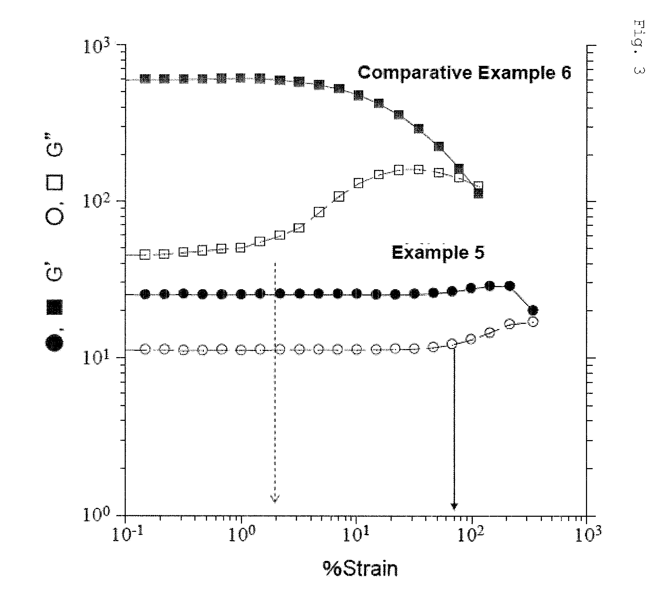

Fig. 3 shows the examples of strain sweep measured in Example 5 and Comparative Example 6. In Fig. 3, G'': the linear strain (%) at the inflection point of loss modulus was obtained (see the arrows in Fig. 3). Taking the sensory evaluation results also into consideration, when the linear strain was 30% or more, more preferable jelly-like feel was considered to be obtained.

### (3) Evaluation of Jelly-like Feel and Smoothness (Sensory Evaluation)

The compositions obtained in the Examples and Comparative Examples, which were used as cosmetic materials, were evaluated by ten subjects in a sensory test.

A sample was supplied to a cylindrical container with a diameter of 5 cm, and the container was gently shaken left and right to evaluate the jelly-like feel. The sample was rated A when ten of the ten subjects strongly felt a jelly-like feel, rated B when eight or more of the ten subjects felt a jelly-like feel, and rated C when seven or fewer subjects felt a jelly-like feel. Regarding the smoothness, the sample was rated A when eight of the ten subjects felt smoothness when the sample was applied to the back of their hands, and the sample was rated B when seven or fewer subjects felt smoothness.

Table 5 shows the results.

**Table 5**

| | | Polymer | Viscosity (mPa·s) | Frequency sweep measurement | | Strain-sweep measurement | Sensory evaluation results | |
|---|---|---|---|---|---|---|---|---|
| | | | | Factor a | Factor b | Linear strain (%) | Jelly-like feel | Smoothness |
| Example | 1 | 1 | 15000 | 0.4 | -0.1 | 40 | B | A |
| | 2 | 2 | 15900 | 0.35 | -0.15 | 100 | B | A |
| | 3 | 3 | 25850 | 0.3 | -0.17 | 120 | A | A |
| | 4 | 4 | 15250 | 0.2 | -0.30 | 70 | B | A |
| | 5 | 5 | 16000 | 0.44 | -0.40 | 70 | A | A |
| | 6 | 6 | 26000 | 0.22 | -0.35 | 220 | A | A |
| Comp. Example | 1 | 4 | 11000 | 0.1 | -0.15 | 35 | B | B |
| | 2 | 5 | 13000 | 0.24 | -0.20 | 70 | A | B |
| | 3 | 6 | 22000 | 0.15 | -0.20 | 170 | A | B |
| | 4 | 4 | 110 | Unmeasurable | Unmeasurable | Unmeasurable | C | - |
| | 5 | 7 | 1200 | Unmeasurable | Unmeasurable | Unmeasurable | C | - |
| | 6 | 8 | 60,000 | 0.07 | 0.22 | 2 | C | A |

## Claims

1. A gel-like composition comprising 0.3 to 3 mass% of an alkyl-modified carboxyl-group-containing water-soluble polymer, 3 to 20 mass% of a polyhydric alcohol, and 0.25 to 5 mass% of an inorganic salt,
the alkyl-modified carboxyl-group-containing water-soluble polymer being
(i) a polymer obtained by polymerizing a (meth)acrylic acid and a C₁₈₋₂₄ alkyl-containing (meth) acrylic acid alkyl ester, or
(ii) a polymer obtained by polymerizing a (meta)acrylic acid, a C₁₈₋₂₄ alkyl-containing (meth)acrylic acid alkyl ester, and a compound having two or more ethylenically unsaturated groups.

2. The gel-like composition according to claim 1, wherein the alkyl-modified carboxyl-group-containing water-soluble polymer is
(ii) a polymer obtained by polymerizing a (meth)acrylic acid, a C₁₈₋₂₄ alkyl-containing (meth) acrylic acid alkyl ester, and a compound having two or more ethylenically unsaturated groups.

3. The gel-like composition according to claim 2, wherein the alkyl-modified carboxyl-group-containing water-soluble polymer is obtained by polymerizing 100 parts by mass of a (meth)acrylic acid, 0.5 to 5 parts by mass of a C₁₈₋₂₄ alkyl-containing (meth)acrylic acid alkyl ester, and 0.001 to 0.1 parts by weight of a compound having two or more ethylenically unsaturated groups.

4. The gel-like composition according to claim 2 or 3, wherein the compound having two or more ethylenically unsaturated groups is at least one member selected from the group consisting of pentaerythritol allyl ethers, diethylene glycol allyl ether, polyethylene glycol diallyl ether, and polyallyl saccharoses.

5. The gel-like composition according to any one of claims 1 to 4, wherein the polyhydric alcohol is at least one member selected from the group consisting of 1,3-butylene glycol, glycerin, diglycerine, propylene glycol, sorbitan, and polyethylene glycols.

6. The gel-like composition according to any one of claims 1 to 5, wherein 0>b and 0.5>a>0.05 in the formula: tan δ = a x frequency (Hz)^{b}, which indicates a relation of loss tangent (tan δ) and frequency (Hz) in a frequency sweep measurement at a temperature of 25°C and a frequency of 1 to 10 Hz.

7. The gel-like composition according to any one of claims 1 to 6, wherein the inorganic salt is an inorganic chloride.
